(19) Europäisches Patentamt
European Patent Office
Office européen des brevets

(11) **EP 4 776 297 A2**

(12) **EUROPEAN PATENT APPLICATION**

(43) Date of publication:
**15.07.2026 Bulletin 2026/29**

(21) Application number: **26151168.7**

(22) Date of filing: **09.01.2026**

(51) International Patent Classification (IPC):
**G16H 20/30** (2018.01)  **G16H 40/40** (2018.01)
**G16H 40/63** (2018.01)  **G16H 50/20** (2018.01)

(52) Cooperative Patent Classification (CPC):
**G16H 40/40; G16H 20/30; G16H 40/63;
G16H 50/20**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: **10.01.2025 US 202563744139 P**

(71) Applicant: **Motif Neurotech, Inc.
Houston, Texas 77006 (US)**

(72) Inventors:
• **Singer, Amanda
Houston, 77006 (US)**

• **Hu, Jia
Houston, 77006 (US)**
• **Commissaris, Elizabeth
Houston, 77006 (US)**
• **Goetz, Steven
Houston, 77006 (US)**
• **Mintch, Landan
Houston, 77006 (US)**
• **Alrashdan, Fatima
Houston, 77006 (US)**
• **Meneau, Phillip
Houston, 77006 (US)**

(74) Representative: **Mewburn Ellis LLP
Aurora Building
Counterslip
Bristol BS1 6BX (GB)**

(54) **PREDICTING OPERATIONAL PERFORMANCE OF MAGNETOELECTRIC FILMS FOR ADAPTIVE CONFIGURATION IN ELECTRONIC DEVICES**

(57)    The present disclosure relates to real-time monitoring and assessment of performance indicators in devices leveraging magnetoelectric (ME) films to predict operational performance-service life, failure risk and acute events of the magnetoelectric films. Predicting the operational performance may involve employing data acquisition techniques to measure various real-time performance indicators including electrical characteristics e.g., voltage, power and/or resonance frequency that can immediately indicate operational status of the ME films, while changes in resistance and capacitance can indicate degradation to assess durability and performance of the ME films. By leveraging data analysis techniques for the measured real-time performance indicators, both gradual degradation and sudden failures may be analyzed that in turn may result in predicting operation performance. Upon predicting the operational performance, alerts or notifications may be generated, via a user-interface for corrective actions and timely interventions, enhancing performance of devices utilizing ME films.

FIG. 1

Processed by Luminess, 75001 PARIS (FR)

EP 4 776 297 A2

## Description

BACKGROUND

Magnetoelectric materials are characterized by the ability to convert magnetic energy into electrical energy and vice versa, making these suitable for various technological applications including medical devices, wireless power transfer, Internet of Things (IoT), aerospace applications, environmental sensors and energy harvesting solutions. Recently, there has been a focus on magnetoelectric (ME) films, particularly for compact and efficient designs (e.g., of the order of 1 cm). Due to their functional capabilities, magnetoelectric films are widely utilized in applications such as sensors, actuators, energy harvesting devices, spintronics, magnetoelectric antennas, ultra power logic devices and high energy density capacitors. Additionally, the ability to control electric properties with magnetic fields and vice versa has led to the exploration of ME films in various medical applications including targeted drug delivery, wireless brain stimulation, tissue regeneration, cancer treatment, molecular-level rapid diagnostics, neural activity recording, and non-invasive bone healing through advanced sensor technologies.

The integration of magnetoelectric films in medical and industrial applications presents promising advancements; however, several challenges may limit their effectiveness. For example, issues such as biocompatibility, stability, and durability under physiological conditions can significantly impact the reliability of devices that utilize these films. Furthermore, design and manufacturing difficulties-including the selection of suitable materials and maintaining operational limits for temperature, magnetic field strength, and electric field strength-can lead to degradation and structural failures. As ME films are increasingly employed in high-stake applications like medical implants and aerospace components, the consequences of these challenges may become more pronounced. Failures in ME films can disrupt vital functions, leading to severe implications for subject safety or operational integrity. Common failure modes may include mechanical stress-induced cracking, thermal fluctuations, chemical degradation from corrosive substances, and electrical overload leading to permanent loss of properties, all of which can compromise performance.

Additionally, devices leveraging ME films may face a challenge in maintaining consistent power delivery due to unpredictable nature of energy demands and the potential degradation of ME films over time. For example, an implant may need more power to stimulate a nerve or a higher voltage to store charge in a capacitor. Moreover, as these films are used to convert magnetic fields into electrical energy, their efficiency can decrease, leading to variations in voltage output. Efficiency may also decrease when one or more ME films are damaged in a device leveraging multiple ME films. This can impact the ability of the device to deliver the required therapeutic energy, particularly in long-term use. Furthermore, changes in the operating state, such as fluctuating power requirements or voltage levels, may further complicate energy management.

SUMMARY

Certain aspects and features of the present disclosure relate to predicting operational performance of the magnetoelectric (ME) films that are integrated in an electronic device by assessing performance indicators of the magnetoelectric films. This predictive capability may facilitate timely interventions, enabling accurate monitoring and maintenance of the current state of the ME films. Subsequently, a configuration of ME films may be dynamically adapted to best meet energy demands, thereby increasing potential of ME films in various applications. The operational performance may include service life, failure risk and/or acute events of the ME films, predicted by monitoring performance indicators that may reflect a current state of the magnetoelectric films. The performance indicators e.g., power output, voltage and/or resonance frequency may be considered as immediate indicators of the operational status of the ME films, while changes in other electrical properties such as resistance and capacitance can indicate degradation. The disclosed techniques may include collecting real-time data associated with one or more performance indicators of a magnetoelectric film integrated in the electronic device, where the magnetoelectric film is configured to supply power to the electronic device. The collected real-time may be processed by leveraging one or more data analysis techniques (e.g., machine-learning techniques or statistical methods) to predict operational performance of the magnetoelectric film.

The processing of the real-time data may include identifying trends representing slow or gradual changes in the one or more performance indicators based on historical data and the real-time data. The historical data may include past operational records of the same or similar magnetoelectric films under normal operations and/or controlled conditions (e.g., controlling applied magnetic field, applied voltage or temperature), also termed herein as templates. For each performance indicator, deviations of the real-time data from corresponding baseline values may be computed. The baseline values may be derived from values of the one or more performance indicators at a manufacturing time of the magnetoelectric film, a reference or template magnetoelectric film, stress-test outcomes or long-term aging studies of the similar magnetoelectric films. The baseline values may represent quantitative measurements based on historical data, testing results, device safety limits and/or manufacturer specifications. These baselines may serve as reference points against which the operational performance of the magnetoelectric film can be assessed dynamically to determine whether the performance of the magnetoelectric film aligns with the expected outcome.

Based on the computed deviations and the identified trends, the service life and/or the failure risk of the magnetoelectric film may be predicted. The service life of the magnetoelectric film may refer to the duration during which the film can operate effectively while maintaining its intended performance characteristic (e.g., voltage, current or power). Failure risk may refer to a predicted risk within one or more defined time periods, for example, a predicted probability that the ME film currently is, or will be failing at a given future time point. For predicting the failure risk, the identified trends against baseline values, predefined thresholds or acceptable performance levels, may be evaluated. If one or more performance indicators approach or exceed these thresholds, it may suggest the failure risk. The historical data and templates from same or similar magnetoelectric films (or materials) may establish what constitutes normal versus problematic performance, thereby identifying deviations that signal service life or failure risk.

The predicted service life may be further compared to an established performance criteria (e.g., allowable ranges) given historical use of a specific subject such that the magnetoelectric film remains suitable for its intended therapeutic application. The established performance criteria may specify the subject-specific ranges that may vary depending on subject medical conditions or treatment goals. For example, the minimum or maximum allowable values of the performance indicators that the magnetoelectric film is to produce for effective stimulation of nerve tissues without causing discomfort or harm to the specific subject. Alternatively, the criteria may specify the required energy needs or levels of service.

Additionally, rapid changes for each of the one or more performance indicators may be identified by comparing the real-time data with the corresponding baseline values to detect an acute event, where the acute event corresponds to a fall or an impact of the magnetoelectric film. For example, if the electronic device integrating the magnetoelectric film experiences a fall or impact, the resulting mechanical stress can lead to immediate changes in the magnetoelectric film's properties e.g., as a sharp decline in voltage output or an abrupt shift in resonant frequency. Such changes may suggest physical damage to the film, such as delamination or microcracking, which could compromise its effectiveness. Based on predicting the operational performance, an alert or notification (e.g., failure of the electronic device, an indication for replacement and/or approaching end-of-service) via a user-interface may be generated that indicates status of the magnetoelectric film, including actions related to service life, acute events or failure risks.

In some examples, on prediction of the operational performance, the device leveraging ME film may be reconfigured dynamically. For example, when predicting performance degradation or an increased failure risk, a drive frequency of an external transmitter, configured to transmit power wirelessly to the ME film, may be adjusted dynamically to match the resonance frequency of the ME film, thereby improving power transfer efficiency. Alternatively, an electrical waveform transmitted by the external transmitter may be modified by lowering an amplitude or output voltage and increasing a pulse width.

In some aspects of the present disclosure, performance indicators of the magnetoelectric films may also be measured or assessed to align a transmitter coil relative to the position of the magnetoelectric films for efficient operation of the ME films. For aligning the transmitter coil, which may be wirelessly coupled to one or more magnetoelectric films within a device enclosure-such as a bioimplant (e.g., a neurostimulator or pacemaker) or an electronic device (e.g., a wireless charger)-the magnetoelectric films may be exposed to a magnetic field generated by the transmitter coil. In response, the magnetic field may generate a performance indicator-an electrical output (e.g., voltage, power or current) across the ME films due to magnetoelectric coupling, which induces a mechanical strain. For each magnetoelectric film, the electrical output can be measured that may vary with changes in the alignment of the transmitter coil. Variations in the electrical output of the magnetoelectric films may provide insights into how well the magnetoelectric films are aligned relative to the exposed magnetic field. Therefore, differences in the electrical outputs among the magnetoelectric films can be assessed to adjust alignment of the transmitter coil.

Additionally, the electrical outputs of each magnetoelectric film may be compared with a dynamic threshold that is based on subject-specific energy consumption (or needs) derived from a treatment plan, and/or a historical usage of a subject. Following this technique, an imperfect transmitter coil position may still be acceptable if the energy consumption of the subject is low. Based on the difference between the pair of magnetoelectric films and comparison with the dynamic threshold, indications may be generated in a feedback mechanism via a user interface to reposition the transmitter coil relative to the one or more magnetoelectric films. For instance, if one or more magnetoelectric films produce lower electrical output compared to other magnetoelectric films or the dynamic threshold, this discrepancy can be used to guide the user in repositioning the transmitter coil such that magnetoelectric films receive the magnetic field efficiently. Therefore, the user may be instructed to move the coil involving vertical movements (e.g., up and down), horizontal movements (e.g., left or right) or along other spatial axes, as indicated in the user interface. The direction to move the transmitter coil may be defined with respect to the observer, viewing the user interface.

In some instances, the dynamic threshold may determine how to configure multiple magnetoelectric (ME) films electrically to best meet a subject's energy needs. In medical devices, the multiple magnetoelectric films may be connected in series for higher operating voltage or in parallel for higher peak currents at lower voltages. The configuration can change based on individual ME film performance or the patient's energy demands. Magnetoelectric films may be reconfigured in real-time to maximize efficiency, adapting to varying energy output levels. For example, parallel films might initially provide adequate voltage but may need to be stacked in series over time due to degradation. Alternatively, if an ME film fails catastrophically (e.g., becomes an open circuit), it may be excluded from the series configuration, or the device may switch to a parallel configuration. The configuration decisions may be made by the implant, the headset supplying the magnetic field, or an external programmer, potentially with input from a clinician or technician.

In some embodiments, a system is provided that includes one or more data processors and a non-transitory computer readable storage medium containing instructions which, when executed on the one or more data processors, cause the one or more data processors to perform part or all of one or more methods disclosed herein.

In some embodiments, a computer-program product tangibly embodied in a non-transitory machine-readable storage medium, including instructions configured to cause one or more data processors to perform part or all of one or more methods or processes disclosed herein.

In some embodiments, a system is provided that includes one or more means to perform part or all of one or more methods or processes disclosed herein.

The terms and expressions which have been employed are used as terms of description and not of limitation, and there is no intention in the use of such terms and expressions of excluding any equivalents of the features shown and described or portions thereof, but it is recognized that various modifications are possible within the scope of the invention claimed. Thus, it should be understood that although the present invention as claimed has been specifically disclosed by embodiments and optional features, modification and variation of the concepts herein disclosed may be resorted to by those skilled in the art, and that such modifications and variations are considered to be within the scope of this invention as defined by the appended claims.

## BRIEF DESCRIPTION OF THE DRAWINGS

The present disclosure is described in conjunction with the appended figures:

FIG. **1** illustrates an application example of magnetoelectric (ME) films integrated in one or more medical implants for wireless power transfer.

FIG. **2** shows an example illustration of an equivalent circuit model of a magnetoelectric film when subjected to an external magnetic field.

FIG. **3A** illustrates an exemplary block diagram to assess one or more performance indicators of the magnetoelectric film for predicting operational performance.

FIG. **3B** shows an illustration of monitoring performance indicators of the magnetoelectric film with accelerated aging to estimate service life, detect failure risk or identify acute events.

FIG. **3C** shows an illustration of monitoring the one or more performance indicators of five devices leveraging the magnetoelectric films with accelerated aging in accordance with an example implementation of the present disclosure.

FIG. **4** illustrates an exemplary block diagram for monitoring and adjusting alignment of a transmitter coil with respect to an implant enclosure comprising a plurality of magnetoelectric films.

FIG. **5** illustrates various arrangements of the magnetoelectric films in accordance with some aspects of the present disclosure.

FIG. **6** illustrates exemplary performance curves depicting power output of the arrangements of the magnetoelectric films with different rectifier configurations.

FIG. **7** illustrates an exemplary workflow of predicting the operational performance of the magnetoelectric film by assessing the one or more performance indicators.

FIG. **8** illustrates an exemplary workflow of aligning the transmitter coil with respect to the implant enclosure comprising the plurality of magnetoelectric films to improve performance.

## DETAILED DESCRIPTION

Some embodiments of the present disclosure relate to real-time monitoring and assessment of performance indicators in electronic devices leveraging magnetoelectric films to predict operational performance-service life, failure risk and acute events of the magnetoelectric films. Predicting the operational performance of the magnetoelectric (ME) films may involve employing data acquisition techniques to measure various real-time performance indicators that may include electrical characteristics e.g., voltage, power and/or resonance frequency that can immediately indicate operational status of the ME films, while changes in resistance and capacitance can indicate degradation to assess durability and performance of the ME films. By leveraging data analysis techniques for the measured real-time performance indicators, both gradual degradation and sudden failures may be analyzed that in turn may result in predicting operational performance such as acute event detection, service life and/or failure risk prediction. Upon predicting the operational performance of the ME films, a dynamic and an adaptive energy management approach may be employed. Additionally, or alternatively, alerts or notifications may be generated, via a user-interface for corrective actions and timely interventions, enhancing performance of the electronic devices utilizing ME films.

The dynamic and adaptive energy management approach for the devices utilizing magnetoelectric films may address the challenges of power variability and ME film degradation. The disclosed techniques may involve monitoring (or collecting) real-time data associated with the performance indicators, such as the power delivered to tissue, the voltage output of the films, and the overall system performance. Based on the real-time data, the configuration of the ME films may be dynamically adjusted. For example, reconfiguring the electrical signal transmitted by an external transmitter or adjusting a drive frequency of the transmitter to match a resonance frequency of the ME film. Similarly, when utilizing multiple films in a device, a configuration (or topology) of the ME film may be switched (e.g., connecting ME films in series or in parallel), deploying one or more rectifiers or altering a configuration of multiple rectifiers, to meet energy output demand. Additionally, or alternatively, power may be redistributed (or on failure of an ME film) in such devices by unlinking the ME films or diverting energy to critical functions when necessary, enabling prioritization of high priory tasks (e.g., tissue stimulation). This adaptive approach may enable that the device remains efficient, maximizes longevity, and continues to meet the subject's energy needs even as the system and ME films degrade over time.

Magnetoelectric (ME) films may be composed of one or more layers of - magnetostrictive and piezoelectric - that work together to transfer strain and generate power or perform other functions. However, when delamination occurs, this strain transfer may become inefficient, causing the ME film to lose its functional capabilities. This failure can be triggered by several factors, such as impact or vibration (relative motion), chemical changes e.g., corrosion, water ingress, or the natural aging of materials. To prevent such failures, the techniques disclosed herein monitor characteristics - performance indicators of the ME films, that may change as the ME film degrades. For example, as delamination begins, the power output may drop, and the resonant frequency may shift, reflecting changes in the structure of the ME material or its ability to oscillate effectively. Similarly, corrosion may increase the electrical resistance, and material aging may affect the capacitance of the ME films.

Due to structural characteristics, the operational performance of magnetoelectric (ME) films can be effectively predicted by various other performance indicators. For example, mechanical characteristics, such as Young's modulus, may provide insight into the stress tolerance and stiffness of the ME film, indicating how the film will perform under mechanical loads and how long it can maintain structural integrity. Additionally, tensile strength may assess the maximum stress the ME film can endure before failure. Thermal properties, including thermal conductivity and expansion coefficients, may provide understanding of temperature fluctuations that can impact performance and contribute to material degradation. Surface characteristics, such as roughness and corrosion resistance, may reflect the influence of environmental factors that may lead to gradual wear and deterioration. Furthermore, evaluating wear and friction properties, along with conducting environmental tests and accelerated aging studies, may offer valuable insights into how prolonged use and varying conditions affect the longevity and reliability of ME films.

In some aspects, data acquisition may be performed for performance indicators such as voltage and power in devices (or electronic devices) that integrate magnetoelectric (ME) films. For instance, the voltage peak generated by the ME film can be captured by the device (e.g., an implant or sensors), which may analyze the data locally, store it for future retrieval, or transmit it to an external receiver (e.g., a programmer of implantable devices or headset) for predicting trends and performing data analysis. Alternatively, or additionally, the ring down behavior of the ME film can be monitored either by the device itself or directly by the receiving coil on the headset. While these measurements provide valuable insights, assessing resonant frequency may present challenges. Measuring resonant frequency during active driving by a magnetic field can be difficult, as the ME films continue to oscillate even after excitation ceases. This post-excitation-ring down phase may offer information about the effective resonant frequency of the ME film. Ring down can be measured by counting the number of oscillations above a given noise floor threshold as it returns to equilibrium after excitation. Alternative measures of ring down may include a change from a first ring down oscillation peak to the subsequent peaks (rate of decrease), or the period between ring down peaks (frequency of the ring down). A careful analysis of the oscillations and noise thresholds may enable obtaining meaningful data regarding the condition and potential degradation of the ME films.

Additionally, the data acquisition of performance indicators may be performed during active use of devices. For example, in medical applications such as neural stimulators, real-time monitoring of the voltage delivered to nerve tissues during therapy sessions can provide immediate insights into the functionality or operational performance of the device. A decline in a performance indicator such as voltage output from the historical data or baseline values may indicate degradation of the ME film, prompting immediate attention to prevent compromised therapeutic efficacy. Furthermore, low-power diagnostic checks may be conducted between therapy sessions, utilizing stored energy to assess performance indicators such as resistance, capacitance, and/or complex impedance. Such diagnostic measures can reveal potential structural issues, such as delamination or aging, by identifying shifts in these metrics that deviate from established baselines.

For example, after a period of magnetic or electric stimulation, the device can perform resistance and capacitance measurements by using stored energy to power these assessments. Once the stimulation stops, the device can switch to a diagnostic mode where it evaluates the electrical properties of the magnetoelectric (ME) films. This may involve measuring current flows in response to a specific voltage applied during stimulation to determine resistance. Similarly, capacitance may be measured by applying the same voltage-held during the stimulation-and observing the resulting charge accumulation after stimulation ends. The real-time data (i.e., including one or more performance indicators) acquired from these measurements can then be transmitted to external systems for further analysis. By leveraging one or more data analysis techniques or by comparing the real-time data against baseline values e.g., obtained during manufacturing, which may be stored either within the device or in a centralized database, the device can assess its current operational state. Alternatively, in devices utilizing multiple ME films, performance can be evaluated relative to one another, even in the absence of baseline data. For example, if three out of four films consistently exhibit a specified voltage under the same external conditions, a significantly lower output from a fourth film may indicate potential damage or degradation, thereby providing insights into the health of the ME films and informing necessary interventions.

In addition to medical applications, electronic devices integrating ME films such as devices equipped with vibration sensors utilizing ME films, continuous monitoring can detect shifts in resonant frequency or increases in impedance, indicative of material fatigue or mechanical stress or surface corrosion. This proactive monitoring may enable maintenance personnel to undertake timely inspections, thereby mitigating the risk of unexpected equipment failures thereby minimizing downtime. Similarly, in consumer devices such as smartphones that employ ME films for haptic feedback, real-time monitoring can provide effective performance. Changes in performance indicators below a certain threshold may trigger user alerts, prompting repairs that may enhance user experience and extend device longevity.

To process the acquired real-time data including performance indicators, various data analysis techniques e.g., machine-learning and/or statistical methods, may be employed. The data analysis may identify trends (or gradual changes), abrupt changes or anomalies within one or more performance indicators by examining real-time data in relation to established baselines, thresholds and/or performance standards that may be derived from the historical data, benchmarking results, stress testing and/or aging studies. By employing techniques such as time-series analysis, regression models, and anomaly detection, the analysis can reveal gradual changes, abrupt shifts, or anomalies in performance. This may allow effective forecasting of future performance and classification of operational states, enabling a clear distinction between gradual degradation and acute failures.

Data analysis techniques may reveal patterns and variations in the data, highlighting areas where the current performance diverges from the expected performance based on (for example) historical data, templates, baselines or benchmark ME films. In some examples, fitting an actual performance curve (e.g., voltage, power output, or resonance shifts over time) to one or more predefined baselines (or templates) representing known failure modes may be a preferred, computationally efficient approach. especially in low power implant devices.

Historical data for performance indicators typically comes from extensive datasets gathered from similar ME films under known conditions, including both operational and stress testing scenarios. Baselines and benchmark data (or values) may provide a reference against which real-time performance indicators can be compared to identify any significant deviations. The baseline data may be derived from values of the performance indicators at a manufacturing time, stress-test outcomes or long-term aging studies of the similar magnetoelectric films. The deviations from expected outcomes and identified trends may enable predictions of both the service life and failure risk of the magnetoelectric film. Service life refers to the duration during which the magnetoelectric film can operate effectively, while failure risk may be indicated by identified trends such as consistent voltage decline or rising impedance. Failure risk may refer to a predicted risk within a defined time period, for example, a predicted probability that the ME film currently is, or will be failing at a given future time point. Alternatively, the failure risk may represent a specific point within the service life based on the identified trends (or gradual changes) in performance indicators, e.g., continuous decline in voltage, continuous increase in impedance, or other performance indicators that imply a degradation (or that the device is approaching a failure point). Evaluating these trends against baseline values and/or predefined thresholds may allow for early detection of potential failures. Historical data from similar films may help differentiate normal performance from concerning deviations corresponding to service life or failure risk prediction.

The predicted service life can be further assessed against established performance criteria, which may outline allowable ranges based on the historical usage of specific subjects (e.g., including power needed during a therapy session or the average interval between sessions). These criteria may vary depending on individual medical conditions or treatment goals. For instance, the criteria may define thresholds that constitute the minimum and maximum allowable values of performance indicators necessary for effective nerve tissue stimulation without causing discomfort or harm. Alternatively, these criteria can specify required energy levels or service needs. If the performance indicators of the existing magnetoelectric film exceed or fall beyond the established thresholds or are projected to do so before the subject's next therapy session, a notification indicating the end of its useful service life may be generated through the user interface. This approach may enable tailoring of the service life to the individual subject's needs, thereby maximizing the device's effectiveness and longevity.

In some examples, upon detection of a downgrade in values of the performance indicators (e.g., decrease in power output), a recommendation may be provided to the user suggesting alternative therapy solutions that better fit the downgraded energy budget. As an example, if an ME film within the implant can no longer generate high voltages necessary to drive high current amplitudes of stimulation pulses, an alternate waveform may be suggested with lower peak amplitudes but larger pulse widths or additional pulses that may be more feasible for the degraded to satisfy.

In some other examples, a detected failure mode may require the system to adjust its functioning in other ways to maintain optimal performance. In one example, corrosion may alter a film's resonant frequency such that driving it at is original design frequency no longer yields maximum power. A system, upon detecting such a failure, might shift the drive frequency of the magnetic field to the new post-corrosion resonance and thereby restoring power output. This process may initiate when voltage or power yielded by an ME film is observed to decrease, might proceed with a characterization of resonance via an observation of film ring down (or alternately by sweeping the drive frequency across a range to look for maximum output), and then a reconfiguration of the coil tuning or drive frequency in the headset to again match the optimum frequency for the degraded film.

Furthermore, rapid changes in performance indicators can be identified by analyzing real-time data against corresponding baseline values to detect acute events. Such events may arise from impacts or falls experienced by the device integrating the magnetoelectric film, leading to immediate alterations in the film's properties, such as a sharp decline in voltage output or a sudden shift in resonant frequency from historical data or baseline values. These changes could indicate physical damage, such as delamination or micro-cracking, which may compromise the film's functionality. To communicate the operational performance, alerts or notifications (e.g., failure of the electronic device, need for replacement, or approaching end-of-service) may be generated through a user interface, providing information on the status of the magnetoelectric film and actions regarding service life, acute events, or failure risk. For example, an action may include generating immediate alerts for users or healthcare providers to assess the device's condition and recommend inspections or replacements. Additionally, initiating diagnostic checks can evaluate performance degradation, enabling timely interventions and adjustments to treatment plans to maintain patient safety and device efficacy.

In some aspects of the present disclosure, techniques may be provided for monitoring and adjusting the alignment of a transmitting coil relative to the one or more magnetoelectric films. When the transmitter coil is activated, the output (e.g., voltage, current or power) of each ME film positioned in an implant enclosure may be measured. Upon identifying a misbalance in the ME film outputs (e.g., power or voltages), a real-time feedback mechanism may be generated in which the user may be prompted via a coil alignment interface to adjust the position of the transmitter coil e.g., including vertical movements (e.g., up and down), horizontal movements (e.g., left or right) or along other spatial axes, as indicated in the user interface. Voltage comparison between multiple films in the implant enclosure may provide real-time guidance for the user, for example, if one or more ME films have significantly lower output, below a certain threshold or compared to the other ME films or, it may serve as an indication that the transmitter coil is misaligned. In some aspects, the thresholds for comparing the outputs of ME films may be dynamically adjusted for adequate coil alignment based on historical data or medical needs of an individual subject. The dynamic adjustment of thresholds can make the medical devices or implant adaptable to varying energy needs, potentially improving both subject outcomes and performance of the medical implants or devices.

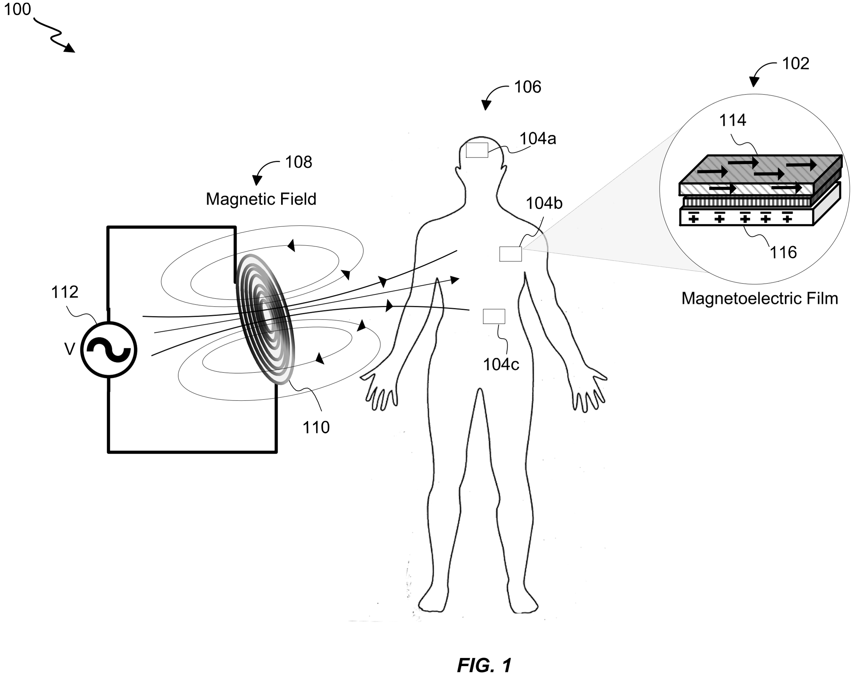

FIG. 1 illustrates an example application of magnetoelectric (ME) films 102 integrated in one or more medical implants such as 104a, 104b and 104c for wireless power transfer. Magnetoelectric materials may be leveraged in harvesting energy from ambient sources such as vibrations, mechanical movements, and magnetic fields that may be utilized for powering low-power electronic devices in remote or inaccessible locations. ME-enabled wireless power transfer may be leveraged for diverse applications eliminating wired connections, for example, ME materials may facilitate wireless charging in consumer electronics (e.g., smartphones, smartwatches, and other portable devices), industrial automation (e.g., robots and electric vehicles), Internet of Things (IoT) and smart cities for deployment of wireless sensors for autonomous environmental monitoring or infrastructure management. Additionally, certain ME materials may be biocompatible and responsive to external stimuli that can be effective to use in biomedical applications such as therapeutic ultrasound, drug delivery systems, tissue engineering i.e., for developing smart biomaterials for tissue regeneration and neural interfaces. ME-based ultrasound devices can generate acoustic waves for non-invasive medical imaging and therapeutic treatments. ME materials can also enhance drug delivery systems by enabling precise control over drug release mechanisms using magnetic and electric fields.

Medical devices, e.g., implants 104a, 104b or 104c within or on a human body 106 may require reliable and continuous power sources without the need for invasive procedures to replace batteries. Magnetoelectric films 102 that combine properties of magnetostrictive layers 114 and piezoelectric layers 116 may offer a solution by efficiently converting external magnetic fields into electrical energy, thereby providing a sustainable power supply for devices e.g., pacemakers, insulin pumps, and neural stimulators. Wireless power transfer may increase patient comfort by eliminating the need for frequent surgeries, reliability and durability, thus enabling uninterrupted operation of the medical devices. For powering the implants 104a, 104b and 104c, an external transmitter coil 110 connected to a voltage source V 112 may generate the magnetic field 108. The one or more magnetoelectric films 102 embedded in the implants 104a, 104b and 104c, within the vicinity of magnetic field 108, may experience this field as it penetrates through the human body 106. The magnetic field 108 may induce a change in magnetization of the magnetostrictive material 114 (shown by '→' in FIG. 1) that may lead to a mechanical deformation or strain in the material. In response to this deformation, an electric field and a corresponding voltage may be induced in an adjacent piezoelectric layer 116 (shown by '+' and '-'). This voltage may be then used (e.g., via electrodes) to power the implants (104a, 104b or 104c) or charge an internal energy storage device, eliminating the need for physical connections or battery replacements. In some examples, the generated electric field from piezoelectric layer 116 may further wirelessly interact with the device in its vicinity.

For medical applications, one or more magnetoelectric films 102 may be arranged within a hermetic or near-hermetic enclosure. This enclosure may be designed for compatibility with implantation or insertion into the body, minimized invasiveness, and miniaturization. ME films 102 may be considered particularly suitable for this application because they can have high output (e.g., >1mW) at small sizes (e.g., or the order of cm). To improve operational performance of the ME films, factors such as the ME film's geometry-including height (or height-to-width ratio), thickness, and orientation relative to incident magnetic fields may be considered. These factors can influence performance indicators such as power output, resonant frequency, and/or voltage. Monitoring these performance indicators over time may help estimating the service life of the ME films, detection of acute events and predicting failure risks. By analyzing changes in these performance indicators, it is possible to forecast when the ME films 102 may fail to meet operational requirements, thus facilitating timely maintenance or replacement to ensure continuous effective performance.

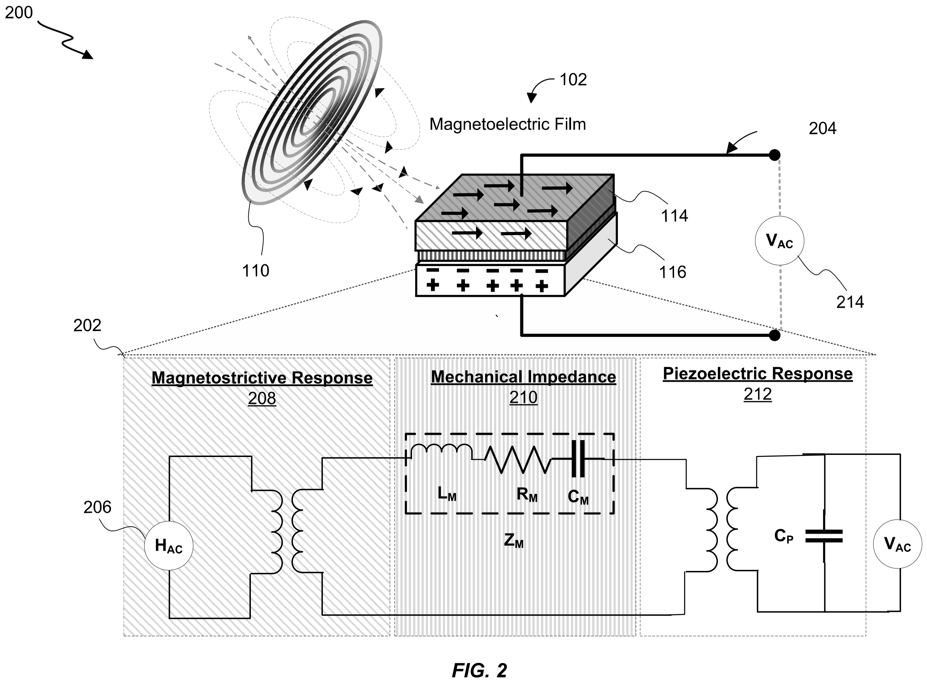

FIG. 2 shows an example illustration 200 of an equivalent circuit model 202 of a magnetoelectric (ME) film 102 when subjected to an external magnetic field 108. The working principle of ME films 102 is the magnetoelectric coupling, where the external magnetic field 108 generated by a transmitter coil 110 induces an electrical response and vice versa. The geometry of ME films 102 typically includes a layered structure combining one or more layers of magnetostrictive material 114 and one or more layers of piezoelectric material 116. This composition may generate magnetoelectric effect under the influence of external magnetic field 108. The magnetostrictive layer 114 is typically made of metallic materials with high permeability such as iron, nickel, boron or their alloys e.g., Metglas, Terfenol-D, which exhibit changes in dimension in response to the applied magnetic field 108. The magnetostrictive layer 114 may experience changes in strain due to application of magnetic field 108. This strain can induce a mechanical stress within the magnetostrictive layer 114 that may be transferred to an adjacent piezoelectric layer 116 comprising of materials such as lead zirconate titanate (PZT), polyvinylidene fluoride (PVDF), or composites of these material. In response, piezoelectric material 116 may generate an electric field and a corresponding voltage $V_{AC}$ 214 (e.g., across electrodes 204). The amplitude of the voltage ($V_{AC}$) can be modulated by shifting the frequency of the applied magnetic field 108.

Apart from magnetostrictive 114 and piezoelectric 116 layers, ME films 102 may include additional layers or components depending upon the specific design and application. These additional layers or components may be incorporated to enhance structural integrity, electrical conductivity or for providing protective coating. For example, buffer layers for adjusting interference between different materials or a substrate layer providing mechanical support, stability, electrical isolation and a foundation upon which the magnetostrictive 114 and piezoelectric 116 layers may be deposited. Moreover, ME film 102 may include electrodes 204 that are typically made of conductive materials such as metals (e.g., gold, silver) or conductive polymers. These electrodes 204 may be attached across ME films 102, as illustrated in FIG. 2, for applying electric signal to the piezoelectric layer 116 and/or extracting electric signals generated by the piezoelectric effect.

In equivalent circuit model 202, $H_{AC}$ 206 may represent the amplitude of the applied magnetic field 108 generated by the transmitter coil 110 that is connected to the voltage source V 110. The equivalent magnetic field $H_{AC}$ 206 may induce the elastic excitation or mechanical stress (used interchangeably herein) in the magnetostrictive layer 114 through a magnetostrictive response 208, which may depend on thickness of the magnetostrictive layer ($t_m$). Losses in the elastic excitation may be represented by an equivalent mechanical impedance $Z_M$ 210 (alternating current (AC) equivalence of resistance $R_M$, inductance $L_m$ and capacitance $C_M$). Effective interface adhesion may assist in transferring strain from the magnetostrictive layer 114 to the piezoelectric layer 116, for improved voltage output and resonance frequency.

Additionally, for generating the output voltage ($V_{AC}$) 214 that may power devices or medical implants, the elastic excitation may be converted into an electric field ($E_{AC}$) through piezoelectric response 212, and the capacitance of the piezoelectric layer $Cp$. The piezoelectric response 212 may be influenced by factors such as thickness of piezoelectric layer ($t_p$), which impacts the voltage and overall power output.

The typical design of ME films 102 contemplates one or several generally rectangular films that may be positioned vertically or horizontally in a medical device enclosure (aligned with the generally symmetric magnetic field created by a single coil). One or more techniques are described herein for monitoring different characteristics or performance indicators of the ME film 102 such as power output, voltage, resonant frequency and other electrical properties (e.g., resistance and/or capacitance) to estimate operational performance e.g., service life, acute events or failure risks of the ME films 102. As the ME films age or experience degradation due to factors like layer delamination, corrosion, or material aging, these performance indicators may also change. By monitoring and analyzing trends in these attributes, it is possible to estimate the remaining service life of the ME films 102, predict failure risks or detect acute events. This predictive analysis may enable the generation of alerts or notifications to relevant stakeholders, such as health practitioners, clinicians, and patients, facilitating timely intervention and maintenance for continued effectiveness and reliability of medical implants or devices.

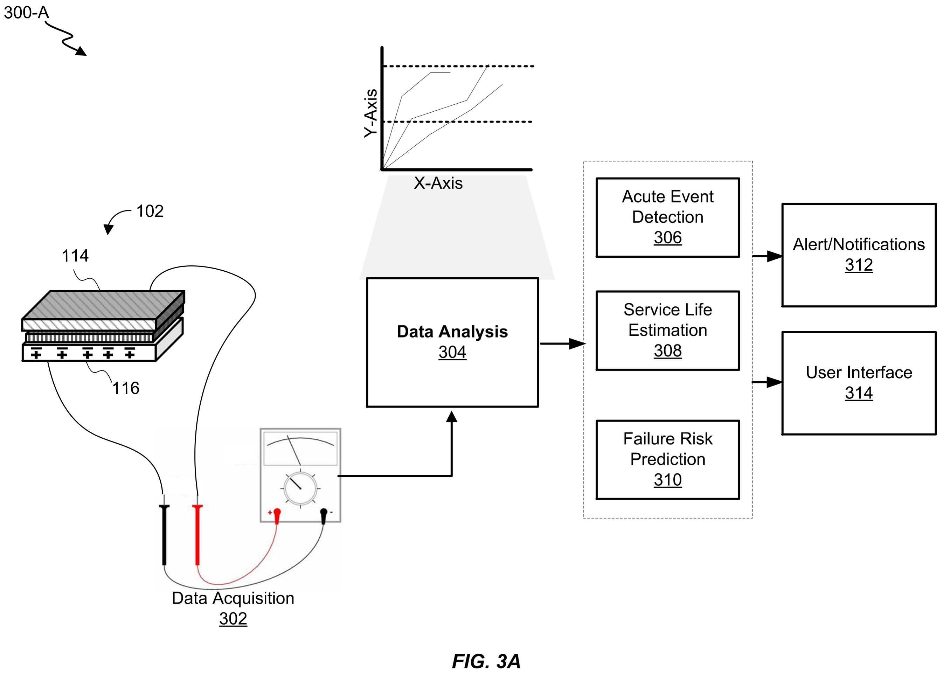

FIG. 3A illustrates an exemplary block diagram 300-A to monitor performance indicators of the magnetoelectric films in real-time. The disclosed techniques may detect slow and/or rapid changes in performance indicators of the magnetoelectric film to predict operational performance including acute event detection 306, service life estimation 308, failure risk prediction 310 and/or generate alerts or notifications 312 for corrective actions. The data acquisition 302 of real-time data may be performed by collecting the performance indicators of the ME film 102 such as voltage, power, resonant frequency, and other possible electrical characteristics e.g., impedance, resistance or capacitance. The data acquisition 302 may involve direct measurements of real-time data (including performance indicators) during therapy sessions (active use), for example, when an implant or a medical device such as a pacemaker or a neural stimulator is delivering therapy (e.g., generating pulses or signals).

Alternatively, or additionally, the medical device integrating one or more ME films 102 may use stored energy to perform diagnostic checks between therapy sessions. These checks may represent low-power measurements to track the health or state of the ME films 102 over time, without needing full operation of the medical device. For example, a magnetic or electric field may be briefly applied to the ME film 102 for measuring the resonant frequency or the impedance of the magnetoelectric film 102. If the resonant frequency is shifting or impedance is increasing in reference to the corresponding historical data, this may suggest that the ME film 102 is degrading, such as through delamination or aging. Ring-down oscillations-the behavior of ME films 102 after the applied field is removed- may also be measured to detect changes in physical structure of the ME films 102. Magnetoelectric film 102 may be subjected to various stresses that can lead to degradation over time, such as delamination, chemical changes, corrosion, material wear, or mechanical impact. Performance indicators from these magnetoelectric films 102 may provide an insight to the current state of the magnetoelectric films 102 and future performance, including failure risks or acute events.

In some examples, performance indicators (PIs) of magnetoelectric (ME) films can be measured using a variety of devices and techniques tailored to their scale, whether for miniaturized bioimplants or larger applications. High-precision power meters and oscilloscopes may be employed to measure peak power output in miniaturized ME films, with specialized instruments for micro-scale measurements. For instance, a wireless power meter can assess power output from outside the body by utilizing transmitter and receiver coils. Additionally, electromagnetic probes may detect the electric or magnetic fields generated by the ME film and convert these into power measurements. To measure resistance, precision digital multimeters and four-point probe techniques are utilized. For resonance frequency, methods may include calibrated network analyzers, frequency counters, and optical techniques such as laser Doppler vibrometry. The ring-down oscillator technique may also be valuable for measuring resonance by evaluating the decay profile of oscillations after excitation.

After the data acquisition 302, data analysis 304 may be performed by processing the measured performance indicators to identify patterns, trends and changes in the characteristics of the ME films 102. To evaluate performance indicators of the magnetoelectric films 102 effectively, a range of data analysis techniques 304, including machine-learning and statistical methods, can be employed. These techniques may leverage real-time performance indicators alongside historical data from the magnetoelectric films 102, such as past operational records (e.g., from earlier instances of the same or similar magnetoelectric film under normal operations), templates or baseline results (i.e., performance indicators collected at the time of manufacture or under controlled conditions to establish performance standards), stress-test outcomes, and long-term aging studies (i.e., performance indicators collected over extended periods of the similar ME films to monitor the changes as the films age). The benchmarking results, i.e., performance indicators measured at the time of manufacture may establish baseline values for the ME films, providing reference points for future analysis. By comparing real-time performance indicators with these initial benchmarks, stress tests and/or long-term aging studies, data analysis techniques 304 can accurately identify trends, detect deviations, and predict potential failures.

The trends or slow changes can indicate gradual degradation mechanisms over time such as corrosion or material aging. Based on the observed trends, the disclosed techniques may estimate the service life, that is how much longer the ME films can perform adequately before failing or degrading significantly. This may involve evaluating whether the current state of the ME films meets the performance standards for their intended use. In addition, the predicted service life may also be evaluated considering individual subject data and how the ME films have performed in the past for that specific subject, helping to tailor predictions and service requirements based on personalized usage patterns.

Data analysis techniques 304 may include time-series analysis that is generally used to detect trends and patterns over time, for example, moving averages and exponential smoothing can help highlight gradual changes in performance indicators, making it easier to identify trends that may signify slow degradation, such as material aging or corrosion. For advanced analysis, statistical models such as ARIMA (autoregressive integrated moving average) can be used to forecast future performance and detect deviations from expected trends. Machine-learning algorithms, particularly supervised learning models, e.g., regression models, including linear and polynomial regression, can be used to predict normal performance and detect deviations. Classification algorithms, such as decision trees and support vector machines, can be trained on historical data to categorize performance states and predict failures based on detected patterns.

Unsupervised learning techniques such as clustering and anomaly detection may be useful for identifying outliers and sudden changes in the data that may indicate acute events. Techniques such as k-means clustering can group similar performance profiles, while algorithms such as Isolation Forest or one-class SVM can isolate anomalies from the normal operating range. Additionally, ensemble methods, which combine multiple machine-learning models, can improve prediction accuracy and robustness. For instance, combining decision trees and support vector machines in an ensemble can provide a better understanding of performance trends and potential failures.

Data analysis 304 may involve comparing the real-time data (i.e., including performance indicators collected from the data acquisition 302) to historical data, baselines or benchmark ME films, identifying deviations from the expected performance. Historical data, baselines, benchmarks or threshold values for the performance indicators typically come from extensive datasets gathered from similar ME films under known conditions, including normal operation and stress testing scenarios. These benchmarks may provide a reference against which real-time performance indicators can be compared to identify any significant deviations. For example, tracking the voltage output or resonant frequency over time can indicate wear or failure. Similarly, the data analysis 304 may detect rapid changes in ME film properties that may indicate an acute event, such as an impact or sudden stress to the film (e.g., a fall). A sudden drop in voltage or a sharp shift in resonant frequency may trigger the detection of acute events, differentiating between slow degradation and acute or unexpected failures.

Leveraging the data analysis techniques 304, an assessment can be made regarding the remaining service life 308 of the magnetoelectric (ME) films. This assessment can be conducted by monitoring gradual degradation or trends in performance indicators to predict the point at which the ME film 102 will no longer fulfill the operational requirements for therapeutic application. For instance, if performance indicators, such as the voltage output of the ME film 102, shows a consistent decline, the remaining time before the ME film falls below a critical performance threshold can be estimated. The performance thresholds may be derived from the historical data including the stress testing, long-term aging studies and/or the benchmark results. Utilizing data analysis 304, failure risk prediction 310 may be executed by trending performance data and comparing it against baseline or reference films.

Upon identifying that the performance of the ME films 102 is approaching or surpassed a critical threshold, alerts or notifications 312 may be generated. These notifications could indicate various statuses, including nearing end-of-service, device failure, or the need for replacement. Such notifications may be directed to relevant stakeholders, including health practitioners, clinicians, and patients, to facilitate timely intervention and necessary actions. The disclosed techniques may provide real-time updates, via a user-interface 314, regarding status of the ME films by displaying current trends, predicting remaining service life and any alerts or notifications 312 related to acute events to take actions based on the acquired data, such as scheduling maintenance or preparing for the device replacement.

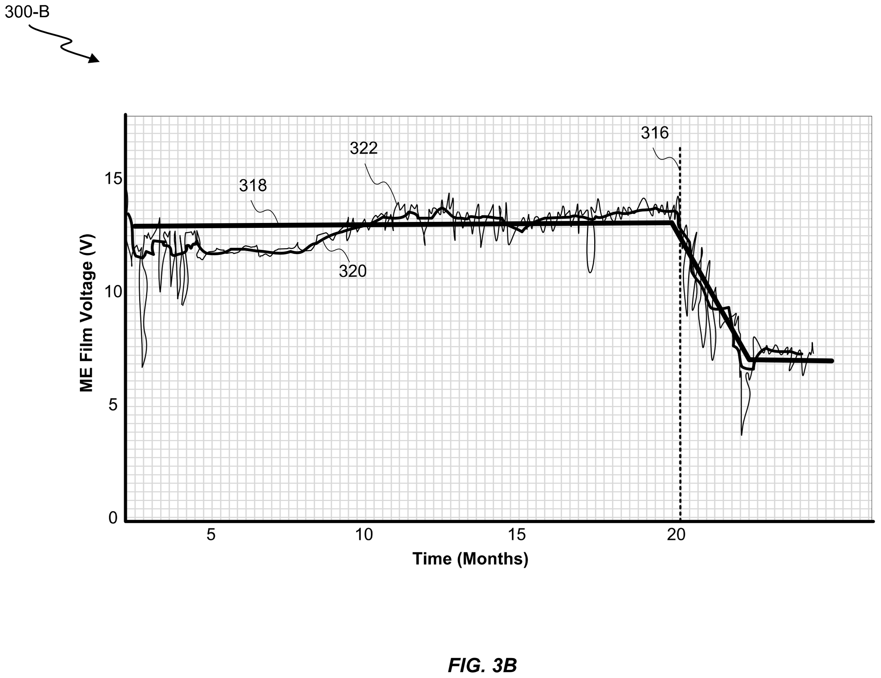

FIG. 3B shows an illustration of monitoring performance indicators of a magnetoelectric (ME) during accelerated aging to estimate service life, detect failure risks or identify critical events. The graph 300-B depicts voltage performance of the ME film collected over time, where a fluctuating line 322 represents real-time voltage data, solid line 320 represents a centered 10-point moving average of the real-time voltage data for trend analysis, and bold line 318 serving as a baseline operational template delineating the states of the ME film as operating normally, degrading or degraded. Comparing real-time voltage performance curve 322 with baseline operational template 318 may enable the detection of performance deviations indicative of aging or failure.

The real-time voltage data curve 322 demonstrates a failure mode attributed to corrosion after approximately 20 months, as indicated by a decline in voltage output at 316. This voltage decline is accompanied by a shift in resonance frequency from $f1$ to $f2$ (where $f2 < f1$). To counteract this voltage decline, a drive frequency of the (external) transmitter coil 110 that is used to power ME film 102 can be dynamically adjusted from $f1$ to $f2$. By continuously tracking voltage trends in real-time, this monitoring approach may provide a robust means of identifying degradation patterns, enabling proactive measures to be taken for failure mitigation and functional lifetime extension of the ME film.

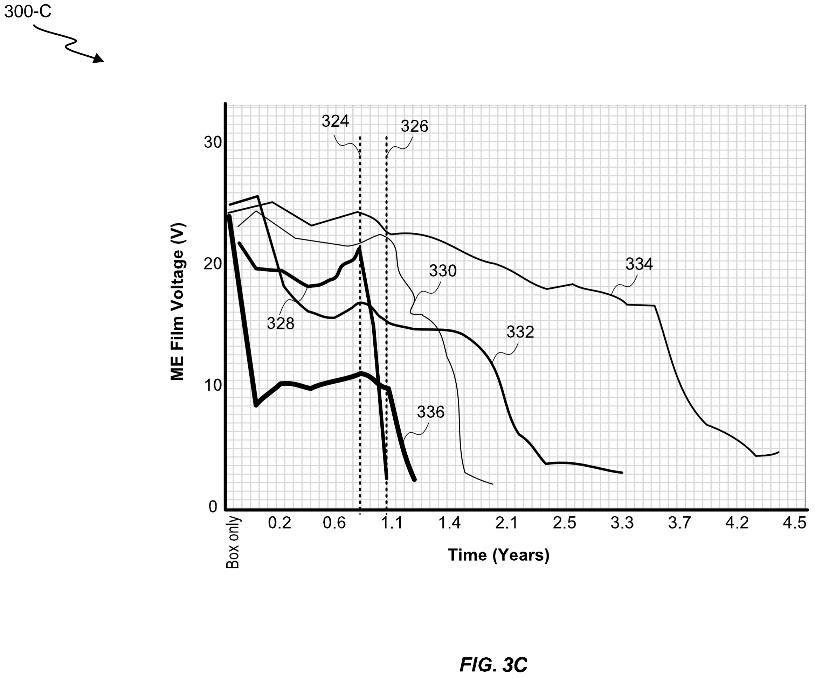

FIG. 3C shows an illustration of monitoring performance indicators of five devices leveraging ME films during accelerated aging in accordance with an example implementation of the present disclosure. The performance indicator curves 300-C depict output voltages of ME films (in volts) with time (in years). Along the time axis (x-axis), the starting point, labeled "box only," represents a time before aging begins. A sharp decline in voltage output indicates potential failures; for instance, voltage curve of Device 1 328 and Device 3 330 show declines at 0.6 years (at 324) and 1.1 years (at 326), respectively. The subject may then be proactively alerted (e.g., via a notification on a GUI) to consult a clinician or healthcare provider for timely replacement, preventing a loss of therapeutic function.

Similarly, voltage curves of Device 2, Device 4, and Device 5 depict slow degradation process indicating corrosion. These voltage curves from these three ME films yield useful power and may be assessed periodically against a dynamic threshold to determine if the device requires reconfiguration for continued operation. For device reconfiguration or to maintain continued operation, the driving frequency of the transmitter coil 110 may be shifted to restore voltage output or stimulation parameters may be adjusted to match the degraded power budget.

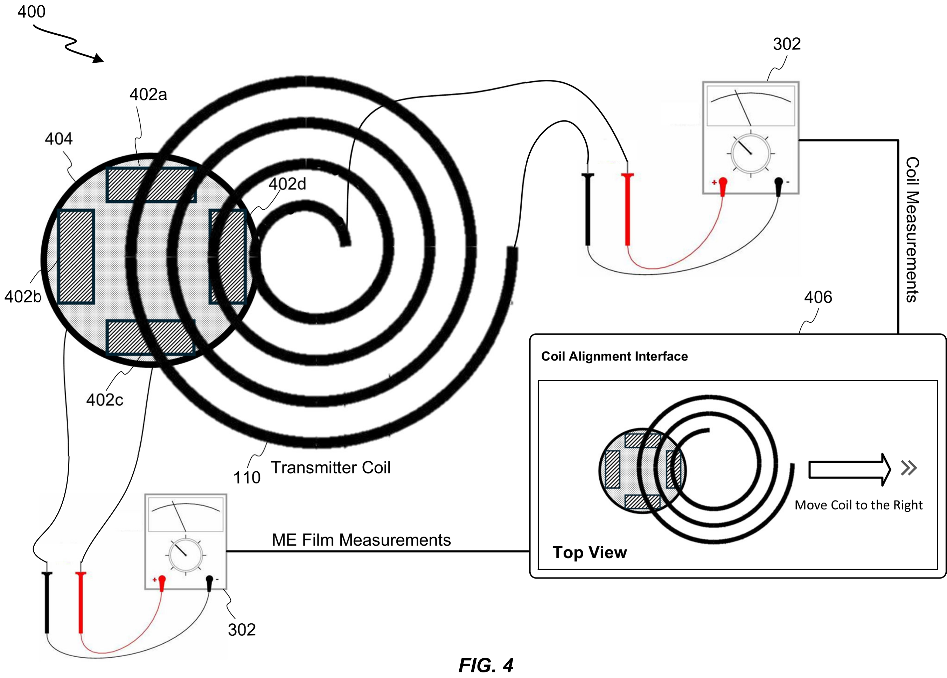

FIG. 4 illustrates an exemplary block diagram 400 for monitoring and adjusting the alignment of a transmitter coil 110 with respect to an implant enclosure 404 comprising a plurality of magnetoelectric (ME) films 402a, 402b, 402c, and 402d. The transmitting coil 110 may generate a magnetic field intended to interact with the ME films in the implant enclosure 404. The alignment of the transmitter coil 110 relative to the ME films may influence the performance indicators such as power transfer, peak power and the voltage generated by the ME films. When the ME films in the implant enclosure 404 are exposed to the magnetic field generated by the transmitter coil 110, the ME films may convert the magnetic field into measurable electric outputs (e.g., voltage, current or power). These outputs may vary as the alignment of the transmitter coil 110 is varied. In some aspects of the present disclosure, the output from the transmitter coil 110, such as voltage or power, may be measured and compared to the outputs from individual magnetoelectric (ME) films (e.g., 402a). Alternatively, the voltages from different ME films in relation to one another may be assessed. Any discrepancies or differences in these voltage measurements may indicate a misalignment of the transmitter coil relative to the ME films, providing valuable insights into the operational alignment and performance of the system. The difference can then be used to inform a user for coil repositioning to improve magnetic field reception.

The alignment of the transmitter coil 110 may be performed in a feedback mechanism, where the system may provide visual indication via an interface or electrical measurements that may guide the user to adjust the transmitter coil 110. For example, if certain ME films are producing lower voltages, the user may be prompted to adjust the position of the transmitter coil 110. The coil alignment interface 406 may provide specific instructions for this adjustment. If the instruction indicates moving the transmitter coil 110 to the right, this direction is defined with respect to the observer's perspective viewing the coil alignment interface 406. The directional adjustment could involve moving the coil in various dimensions: upward, downward, left, right, or along other axes depending on the layout of the coil alignment interface 406. For instance, moving the transmitter coil 110 to the right means shifting it laterally in the horizontal plane as perceived from the observer's viewpoint. Similarly, vertical movements (upward or downward) or adjustments along other spatial axes may be performed within the three-dimensional alignment framework, guided by the visual cues and instructions provided in the coil alignment interface 406. Additionally, instead of relying on a static threshold, the disclosed techniques may include dynamically adjustment of the threshold based on the historical data, specific treatment requirements, or real-time usage information. The dynamic adjustment may allow for greater flexibility, particularly when a subject's energy needs are lower during a particular session, and less-than-perfect alignment could still be acceptable.

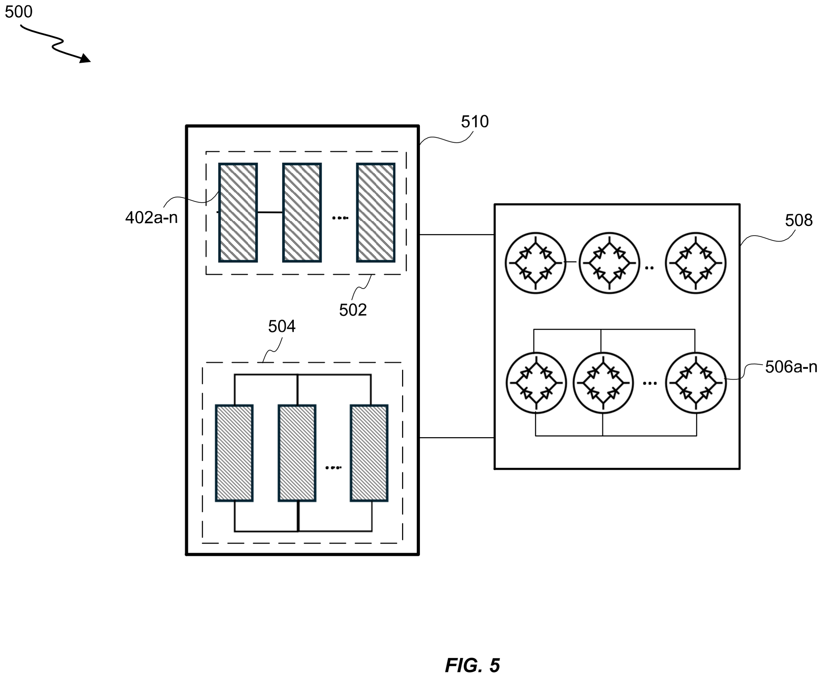

The plurality of ME films enclosed in a medical device can be arranged in various arrangements to optimize the performance of the medical device based on operational requirements or the dynamic threshold. FIG. 5 illustrates various configurations 500 associated with the ME films and rectifier circuits in accordance with some aspects of the present disclosure. For example, ME films can be arranged or configured in series, as shown in 502, resulting in higher voltages (or voltage tolerance), which may be advantageous for storage or handling of higher voltages. However, this configuration may be less efficient under high load conditions due to the limited current capacity, resulting in lower available power. Alternatively, when the ME films 402a-n are configured in parallel, as shown in 504, the current capacity is increased, delivering higher power under load, though the voltage tolerance remains limited by the individual ME films. The ME films 402a-n can be integrated with various configurations of rectifier circuit 508, which may include one or more series or parallel rectifiers 506a-n, either assigned to each individual ME film or shared across the plurality of ME films 402a-n using a single rectifier circuit. Using multiple rectifiers may enhance the performance by decoupling the individual ME films, thereby minimizing the effects of small parameter mismatches between them and providing more consistent operation.

As the optimal tradeoff between power and voltage can vary depending on the operational state of the medical device, it may be advantageous to adapt the configuration of the ME films 402a-n dynamically. For instance, prior to a stimulation event, a series configuration 502 may be preferable to accumulate energy in a capacitor, where the stored energy is proportional to the square of the voltage ($E = \frac{1}{2} * C \times V^2$), and higher voltage values are beneficial. However, during the stimulation event itself, when the device is under high load and the ME film voltages are lower, a parallel configuration might provide better performance by delivering higher power. The device could monitor its operating state through various parameters, such as power input to tissue, the ability to maintain a compliance voltage, or directly from the rectified output of the ME films 402a-n. Based on these measurements, the medical device may dynamically adjust the arrangement of ME films 510 and corresponding rectifier configuration 508 for optimal performance. Additionally, the device may selectively unlink two or more operating ME films and redirect their outputs to different purposes if energy usage is unpredictable. This approach could prevent system failure by reserving energy for critical functions, enabling the device to be operational under varying conditions.

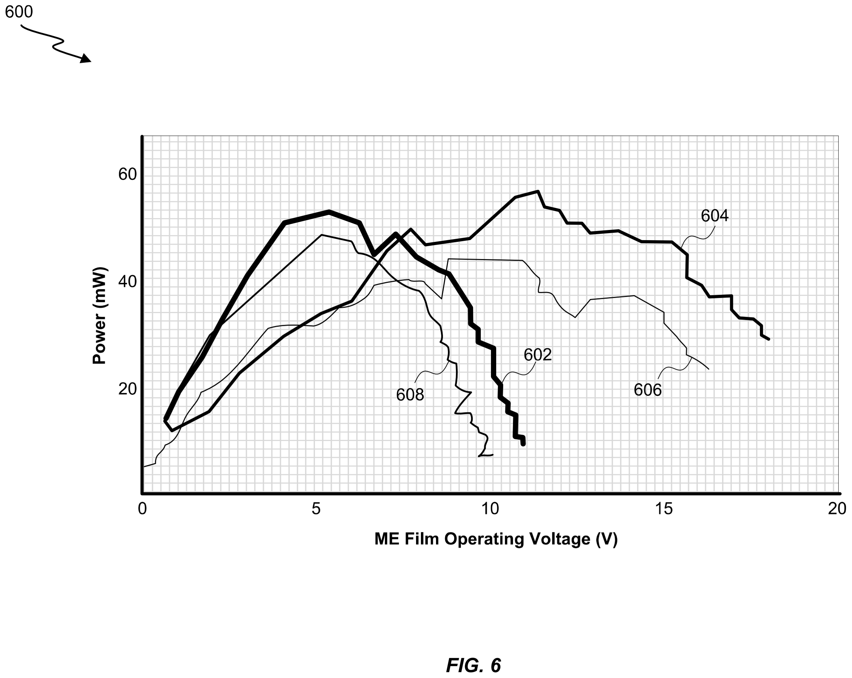

FIG. 6 illustrates exemplary performance curves 600 depicting power output of magnetoelectric (ME) film arrangements 510 with different rectifier configurations 508 e.g., parallel two-rectifiers 602, series two-rectifiers 604, series one-rectifier 606, and parallel one-rectifier 608. Each curve highlights the relationship between ME film operating voltage (x-axis) and the resulting power output (y-axis) in mW (milli Watts), revealing an impact of both the rectifier arrangement and the number of rectifiers on power efficiency. Based on the exemplary performance curves 600, an observation regarding rectifier configuration can be made that series configurations maintain a steady increase in power output with operating voltages showing higher efficiency for wider operating voltage ranges. While, parallel configurations excel in current handling, maintaining higher power outputs in a narrower operating voltage range. Adding more rectifiers improves performance (i.e., higher peak powers and broader operating voltages) in both configurations. For example, series two-rectifier configuration 604 outperforms series one-rectifier by achieving higher power and a broader operating voltage range. The series two-rectifier configuration 604 achieves highest power output among all these configurations, with a peak power exceeding approximately 50mW at moderate operating voltages. The parallel two-rectifier configuration 602 shows a slightly lower peak power compared to the series two-rectifier configuration 604 but maintains higher power for smaller operating voltage. Additionally, parallel configurations, i.e., 602 and 608 show a sharp decline at higher operating voltages.

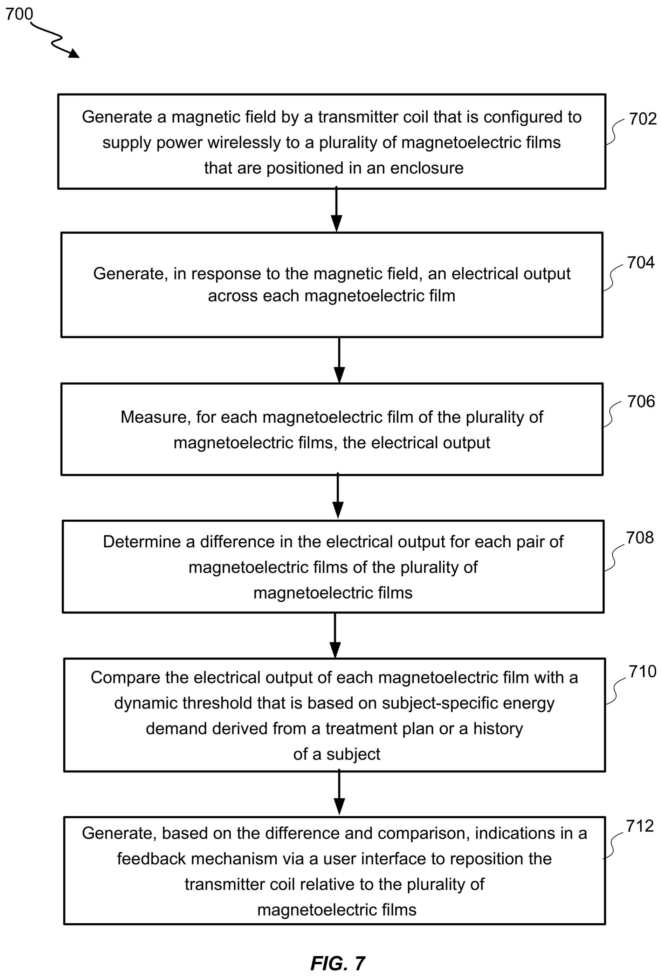

FIG. 7 illustrates an exemplary workflow 700 of aligning a transmitter coil 110 relative to one or more magnetoelectric films to improve performance of the magnetoelectric film 102. The blocks in the exemplary workflow 700 are illustrated in a specific order, while the order can be modified, for example, some blocks may be performed before others, and some blocks may be performed simultaneously. The blocks can be performed by hardware, software, or a combination thereof. At block 702, a magnetic field 108 may be generated by a transmitter coil 110 that is configured to supply power wirelessly to a plurality of magnetoelectric films e.g., 402a, 402b, 402c and 402d that may be positioned in an enclosure 404 (e.g., a bioimplant or an electronic device). At block 704, the magnetic field 108 may generate an electrical output (e.g., voltage ($V_{AC}$) 214, power or current) across the ME films (402a, ..., 402d) due to magnetoelectric coupling, which induces a mechanical strain. The electrical output such as voltage ($V_{AC}$) 214 may be collected via a pair of electrodes 204 mounted across the ME film 102. For each magnetoelectric film 402, the electrical output may be measured, at block 707. These electrical outputs may vary with the alignment of the transmitter coil 110 therefore, differences in the electrical outputs for each pair of magnetoelectric films (e.g., 402a and 402b) may be determined, at block 708.

Additionally, at block 710, the electrical outputs of each magnetoelectric film 402 may be compared with a dynamic threshold that is based on subject-specific energy demand derived from a treatment plan (e.g., nerve stimulation) or a history of a subject. At block 712, based on the difference between the pair of magnetoelectric films and comparison with the dynamic threshold, indications may be generated in a feedback mechanism via a user interface (such as coil alignment interface 406 of FIG. 4) to reposition the transmitter coil 110 relative to the one or more magnetoelectric films (e.g., 402a, ..., 402d). For example, if certain ME films (e.g., 402a and 402b) are producing lower voltages as compared to the dynamic threshold, the user may be instructed to move the coil in a particular direction, as indicated in the coil alignment interface 406. The direction to move the transmitter coil 110 to the right may be defined with respect to the observer, viewing the coil alignment interface 406.

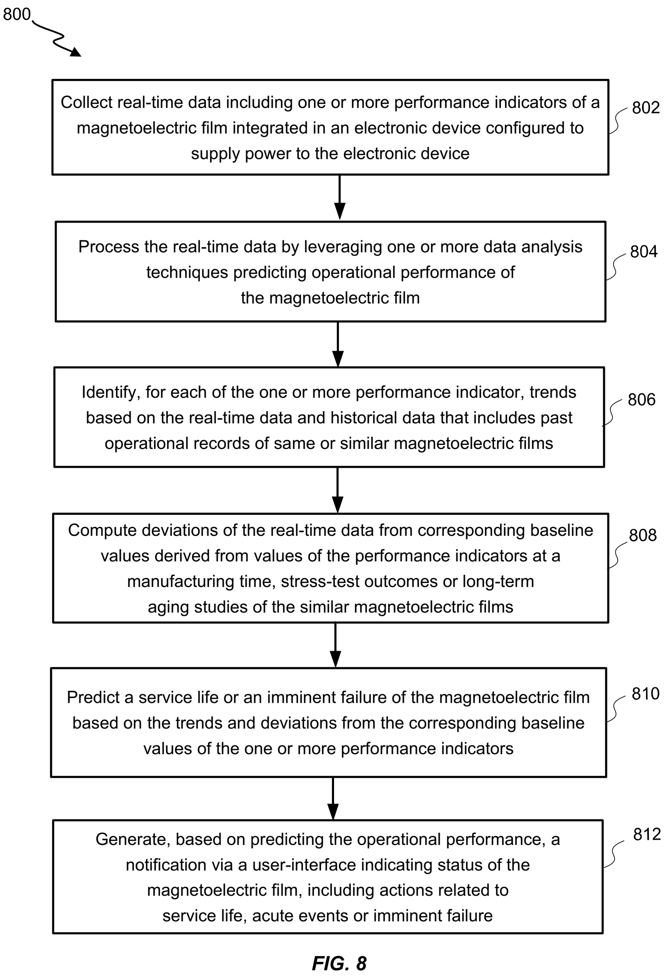

FIG. 8 illustrates an exemplary workflow 800 of predicting operational performance of the magnetoelectric (ME) film 102 by assessing one or more performance indicators. The blocks in workflow 800 are illustrated in a specific order, while the order can be modified, for example, some blocks may be performed before other, and some blocks may be performed simultaneously. The blocks can be performed by hardware, software, or a combination thereof. The performance indicators (e.g.,

power output, voltage and/or resonance frequency or other electrical characteristics) may reflect the current state of the magnetoelectric films. Therefore, the operational performance such as service life, failure risk and/or acute events of the ME films may be predicted by collecting real-time data including one or more performance indicators of a magnetoelectric film 102, at block 802. The magnetoelectric film may be integrated into an electronic device (e.g., a bioimplant or consumer electronics) for supplying power. At block 804, the collected real-time data may be processed by leveraging one or more data analysis techniques such as machine-learning techniques trained on historical data or statistical methods including time-series analysis.

The processing of the performance indicators may include identifying trends representing slow changes in the one or more performance indicators based on the historical data and the real-time data, at block 806. The historical data may include past operational records of same or similar magnetoelectric films under normal operations and/or controlled conditions (e.g., controlling stress, applied voltage or temperature). At block 808, for each performance indicator, deviations of the real-time data from the corresponding baseline values may be computed. These baseline values may be derived from values of the one or more performance indicators at a manufacturing time of the magnetoelectric film, a reference magnetoelectric film, stress-test outcomes or long-term aging studies of the similar magnetoelectric films. The baseline values may represent quantitative measurements based on historical data, testing results, or manufacturer specifications. These baselines may serve as reference points against which the operational performance of the magnetoelectric film can be assessed to determine whether the performance of the magnetoelectric film aligns with the expected outcome.

In addition, rapid changes for each of the one or more performance indicators may be identified by comparing the real-time data with the corresponding baseline values to detect an acute event, where the acute event corresponds to a fall or an impact of the magnetoelectric film. For example, if the electronic device integrating the magnetoelectric film experiences a fall or impact, the resulting mechanical stress can lead to immediate changes in the magnetoelectric film's properties as a sharp decline in voltage output or an abrupt shift in resonant frequency. Such changes may suggest physical damage to the film, such as delamination or micro-cracking, which could compromise its effectiveness.

At block 810, based on the computed deviations and the identified trends, the service life or the failure risk of the magnetoelectric film may be predicted. The predicted service life may be compared to established performance criteria (e.g., allowable ranges) given historical use of a specific subject such that the magnetoelectric film remains suitable for its intended therapeutic application. The established performance criteria may specify the subject-specific ranges that may vary depending on subject medical conditions, treatment goals. For example, the minimum or maximum allowable values of the performance indicators that the magnetoelectric film is to produce for effective stimulation of nerve tissues without causing discomfort or harm to the specific subject. Alternatively, the criteria may specify the required energy needs or levels of service. Based on predicting the operational performance, at block 812, an adaptive energy management approach may be employed. Additionally, or alternatively an alert or notification (e.g., failure of the electronic device, an indication for replacement and/or approaching end-of-service) via a user-interface may be generated that indicates status of the magnetoelectric film, including actions related to service life, acute events or failure risks.

Although specific aspects have been described, various modifications, alterations, alternative constructions, and equivalents are possible. Embodiments are not restricted to operation within certain specific data processing environments but are free to operate within a plurality of data processing environments. Additionally, although certain aspects have been described using a particular series of transactions and steps, it should be apparent to those skilled in the art that this is not intended to be limiting. Although some flowcharts describe operations as a sequential process, many of the operations can be performed in parallel or concurrently. In addition, the order of the operations may be rearranged. A process may have additional steps not included in the figure. Various features and aspects of the above-described aspects may be used individually or jointly.

Further, while certain aspects have been described using a particular combination of hardware and software, it should be recognized that other combinations of hardware and software are also possible. Certain aspects may be implemented only in hardware, or only in software, or using combinations thereof. The various processes described herein can be implemented on the same processor or different processors in any combination.

Where devices, systems, components or modules are described as being configured to perform certain operations or functions, such configuration can be accomplished, for example, by designing electronic circuits to perform the operation, by programming programmable electronic circuits (such as microprocessors) to perform the operation, such as by executing computer instructions or code, or processors or cores programmed to execute code or instructions stored on a non-transitory memory medium, or any combination thereof. Processes can communicate using a variety of techniques including but not limited to conventional techniques for inter-process communications, and different pairs of processes may use different techniques, or the same pair of processes may use different techniques at different times.

Specific details are given in this disclosure to provide a thorough understanding of the aspects. However, aspects may be practiced without these specific details. For example, well-known circuits, processes, algorithms, structures, and techniques have been shown without unnecessary detail in order to avoid obscuring the aspects. This description provides example aspects only, and is not intended to limit the scope, applicability, or configuration of other aspects. Rather, the preceding description of the aspects can provide those skilled in the art with an enabling description for implementing various aspects. Various changes may be made in the function and arrangement of elements.

The specification and drawings are, accordingly, to be regarded in an illustrative rather than a restrictive sense. It can, however, be evident that additions, subtractions, deletions, and other modifications and changes may be made thereunto without departing from the broader spirit and scope as set forth in the claims. Thus, although specific aspects have been described, these are not intended to be limiting. Various modifications and equivalents are within the scope of the following claims.

## Claims

1. A method comprising:

   collecting real-time data associated with one or more performance indicators of a magnetoelectric film that is integrated in an electronic device, configured to supply power to the electronic device, wherein the magnetoelectric film is wirelessly powered by an external transmitter coil; processing the real-time data by leveraging one or more data analysis techniques predicting operational performance of the magnetoelectric film, wherein processing the real-time data includes:

   identifying, for each of the one or more performance indicators, trends based on the real-time data and historical data that includes past operational records of same or similar magnetoelectric films; computing, for each of the one or more performance indicators, deviations of the real-time data from corresponding baseline values that are derived from values of the one or more performance indicators at a manufacturing time of the magnetoelectric film, stress-test outcomes or long-term aging studies of the similar magnetoelectric films; identifying rapid changes for each of the one or more performance indicators by comparing the real-time data with the corresponding baseline values to detect an acute event;

   predicting a service life or a failure risk of the magnetoelectric film based on the trends and deviations from the corresponding baseline values of the one or more performance indicators; and comparing the predicted service life to an established performance criteria given historical use of a specific subject such that the magnetoelectric film remains suitable for its intended therapeutic application; and

   generating, based on predicting the operational performance, a notification via a user-interface indicating status of the magnetoelectric film, including actions related to service life, acute events or failure risk.

2. The method of claim 1, wherein the actions including:

   dynamically adjusting, based on prediction of the operational performance, a drive frequency of the external transmitter coil to match a resonance frequency of the magnetoelectric film; and dynamically adjusting an amplitude and a pulse width of an electrical signal transmitted by the external transmitter.

3. The method of claim 1, wherein the one or more data analysis techniques include a machine-learning model trained on the historical data collected at different time intervals.

4. The method of claim 1, wherein the performance indicators include one or more of: power output, resistance, capacitance, resonant frequency, or voltage of the magnetoelectric films.

5. The method of claim 1, wherein the acute event is detected if the deviations of at least one of the one or more performance indicators are greater than a given threshold.

6. The method of claim 1, wherein the acute event corresponds to a fall or an impact of the magnetoelectric film.

7. The method of claim 1, wherein the notification includes a failure of the electronic device within a predefined time, an indication for replacement, approaching end-of-service.

8. A method including:

   generating a magnetic field by a transmitter coil that is configured to supply power wirelessly to a plurality of magnetoelectric films that are configured in an enclosure;

generating, in response to the magnetic field, an electrical output across each magnetoelectric film of the plurality of magnetoelectric films;

measuring, for each magnetoelectric film of the plurality of magnetoelectric films, the electrical output;

determining a difference in the electrical output for each pair of magnetoelectric films of the plurality of magnetoelectric films;

comparing the electrical output of each magnetoelectric film with a dynamic threshold that is based on subject-specific energy demand derived from a treatment plan or a history of a subject; and

generating, based on the difference and comparison, indications in a feedback mechanism via a user interface to reposition the transmitter coil relative to the plurality of magnetoelectric films.

9. The method of claim 8, further including:
dynamically configuring, based on the dynamic threshold, the plurality of magnetoelectric films in a series configuration.

10. The method of claim 8, further including:
dynamically configuring, based on the dynamic threshold, the plurality of magnetoelectric films in a parallel configuration.

11. The method of claim 8, wherein the plurality of magnetoelectric films is connected to one or more rectifier circuits.

12. The method of claim 11, wherein the one or more rectifier circuits are connected in parallel or in series.

13. The method of claim 8, wherein a magnetoelectric film of the plurality of magnetoelectric films comprising:

at least one layer of magnetostrictive material configured to be magnetized inducing a mechanical strain when the magnetic field generated by the transmitter coil, is applied; and
at least one layer of piezoelectric material on the at least one layer of magnetostrictive material configured to generate the electrical output in response to the mechanical strain from the at least one layer of magnetostrictive material.

14. A system comprising:
one or more data processors, the one or more data processors configured to perform or implement the method of any of claims 1 to 13.

15. A computer-program product including instructions, which when executed by one or more data processors, cause the one or more data processors to perform or implement the method of any of claims 1 to 13.

16. A system comprising:

a plurality of magnetoelectric films configured in an enclosure;
a transmitter coil configured to supply power wirelessly to the plurality of magnetoelectric films, wherein the transmitter coil is configured to generate a magnetic field, wherein in response to the magnetic field, an electrical output is generated across each magnetoelectric film of the plurality of magnetoelectric films; and,
one or more data processors configured to:

measure, for each magnetoelectric film of the plurality of magnetoelectric films, the electrical output;
determine a difference in the electrical output for each pair of magnetoelectric films of the plurality of magnetoelectric films;
compare the electrical output of each magnetoelectric film with a dynamic threshold that is based on subject-specific energy demand derived from a treatment plan or a history of a subject; and,
generate, based on the difference and comparison, indications in a feedback mechanism via a user interface to reposition the transmitter coil relative to the plurality of magnetoelectric films.

17. The system of claim 16, wherein the system is configured to perform the method of any of claims 9 to 13.

**FIG. 1**

EP 4 776 297 A2

**FIG. 2**

FIG. 3A

FIG. 3B

FIG. 3C

**FIG. 4**

EP 4 776 297 A2

**FIG. 5**

EP 4 776 297 A2

FIG. 6

700

| Generate a magnetic field by a transmitter coil that is configured to supply power wirelessly to a plurality of magnetoelectric films that are positioned in an enclosure | 702 |

| Generate, in response to the magnetic field, an electrical output across each magnetoelectric film | 704 |

| Measure, for each magnetoelectric film of the plurality of magnetoelectric films, the electrical output | 706 |

| Determine a difference in the electrical output for each pair of magnetoelectric films of the plurality of magnetoelectric films | 708 |

| Compare the electrical output of each magnetoelectric film with a dynamic threshold that is based on subject-specific energy demand derived from a treatment plan or a history of a subject | 710 |

| Generate, based on the difference and comparison, indications in a feedback mechanism via a user interface to reposition the transmitter coil relative to the plurality of magnetoelectric films | 712 |

*FIG. 7*

800

Collect real-time data including one or more performance indicators of a magnetoelectric film integrated in an electronic device configured to supply power to the electronic device — 802

Process the real-time data by leveraging one or more data analysis techniques predicting operational performance of the magnetoelectric film — 804

Identify, for each of the one or more performance indicator, trends based on the real-time data and historical data that includes past operational records of same or similar magnetoelectric films — 806

Compute deviations of the real-time data from corresponding baseline values derived from values of the performance indicators at a manufacturing time, stress-test outcomes or long-term aging studies of the similar magnetoelectric films — 808

Predict a service life or an imminent failure of the magnetoelectric film based on the trends and deviations from the corresponding baseline values of the one or more performance indicators — 810

Generate, based on predicting the operational performance, a notification via a user-interface indicating status of the magnetoelectric film, including actions related to service life, acute events or imminent failure — 812

*FIG. 8*